# EUROPÄISCHE PATENTSCHRIFT

(11) **EP 1 790 315 B1**
(45) Veröffentlichungstag und Bekanntmachung des Hinweises auf die Patenterteilung: **20.04.2011**
(21) Anmeldenummer: 06024079.3
(22) Anmeldetag: 21.11.2006
(51) Int. Cl.: A61F 2/08

(54) **Vorrichtung zum Verblocken eines Sehnentransplantates**
Apparatus for anchoring autologous tendon
Appareil d'ancrage de greffes de tendons autologues

(30) Priorität: 28.11.2005 DE 102005057111
(43) Veröffentlichungstag der Anmeldung: 30.05.2007
(73) Patentinhaber: Karl Storz GmbH & Co. KG, 78532 Tuttlingen (DE)
(72) Erfinder: Strobel, Michael J., Prof. Dr., 94315 Straubing (DE); Weiler, Andreas, Dr., 13505 Berlin (DE); Berberich, Sascha, 78532 Tuttlingen (DE)
(74) Vertreter: Weller, Wolfgang

(56) Entgegenhaltungen:
- EP-A2- 1 281 376
- WO-A-95/11631
- WO-A-95/25469
- US-B1- 6 379 361
- US-B1- 6 579 295

## Beschreibung

Die Erfindung betrifft eine Vorrichtung zum Verblocken eines Sehnentransplantates in einer Bohrung in einem Knochen, mit einem Element, das beim Gebrauch in die Bohrung einbringbar ist, wobei das Element zwei keilartige Körper aufweist, die in axialer Richtung zueinander verschiebbar sind, wodurch das Element spreizbar ist, wobei das Element, vor einer Spreizung, etwa zylindrisch ausgebildet ist und das Element über einen Schrägschnitt in zwei keilartige Körper aufgeteilt ist.

Eine derartige Vorrichtung ist aus der EP 1281376 A2 bekannt.

Das Element weist zwei keilartige Körper auf, die an den Flächen, über die sie aneinander liegen, mit einer Stufenrastung versehen sind. Ein Sehnentransplantat wird U-förmig in eine Sacklochbohrung im Knochen eingelegt und zwischen die beiden Schenkel des U wird das Element eingeschoben und dann gespreizt. Dazu sind die beiden keilartigen Körper zunächst so axial gegeneinander verschoben, dass der Körper einen kleinen Durchmesser aufweist, der ausreichend ist, damit die axial verschobenen keilartigen Körper zwischen die U-förmigen Schenkel eingeschoben werden können. Dazu ist einer der beiden keilartigen Körper mit einer durchgehenden Öffnung versehen, in die ein Handhabungswerkzeug eingeschoben werden kann. Von einem der keilartigen Körper springt von der Keilfläche eine Zunge vor, die in eine entsprechende Nut an der gegenüberliegenden Keilfläche des anderen keilartigen Körpers eingreift. Zum Spreizen werden die keilartigen Körper axial gegeneinander verschoben, wobei sich der ursprüngliche Außendurchmesser des Körpers vergrößert. Im Endzustand, also nach dem Verschieben der keilartigen Körper zum Spreizen, nimmt der Körper eine etwa zylindrische Form ein. Dadurch ist das U-förmige Sehnentransplantat in der Sacklochbohrung verblockt.

Aus der WO 95/25469 A ist ein Fastener bekannt, um Gegenstände an einem Knochen zu befestigen. Ein stößelartiger Eintreiber dient dazu, ein radiales Spreizen von Bauelementen zu bewirken, die ein Verblocken in der Bohrung bewirken. Der Stößel selbst weist eine Sollbruchstelle in Form einer Einschnürung auf, die dafür sorgt, dass der Stößel über die Sollbruchstelle abgebrochen und nach der Handhabung abgenommen werden kann.

Derartige Sehnentransplantate dienen insbesondere dazu das vordere oder das hintere Kreuzband des Kniegelenkes bei Verletzungen zu ersetzen. Die allgemeine Operationstechnik ist in "Strobel, Michael: Arthroskopische Chirurgie, Springer, 1998 in Kapitel 2, Kniegelenk" ausführlich beschrieben.

Beispielsweise wird zur Verankerung eines Sehnentransplantates im Oberschenkelknochen in diesen, vom Kniegelenk ausgehend, eine Bohrung eingebracht, die einen ersten Abschnitt mit einem ersten Durchmesser zur Aufnahme des Sehnentransplantates aufweist. An diesen ersten Abschnitt schließt sich ein zweiter durchmessergeringerer Abschnitt an, der durch den Oberschenkelknochen bis zu dessen Außenseite reicht. Durch diesen zweiten Abschnitt werden Durchzugfäden, die mit dem Transplantat verknotet sind hindurchgezogen und an der Außenseite durch einen sogenannten Fixationsbutton fixiert.

Aus der US 6,264,694 B1 ist bekannt geworden am Einführende des Sehnentransplantates ein Element in Form einer Kugel vorzusehen, die sowohl mit dem Sehnentransplantat als auch mit den Durchzugfäden verbunden ist.

Der Außendurchmesser dieser Kugel ist so bemessen, dass diese in den ersten Abschnitt der Bohrung mit dem größeren Durchmesser eingeführt werden kann, jedoch nicht in den zweiten durchmessergeringeren Abschnitt eintreten kann. Nach Spannen der Durchzugfäden liegt das kugelförmige Element an der Schulter am Übergang vom ersten durchmessergrößeren Abschnitt der Bohrung zum zweiten durchmessergeringeren Abschnitt an. Diese Kugel verhindert, dass das Sehnentransplantat beim Ziehen an den Durchzugfäden in den zweiten durchmessergeringeren Abschnitt eintritt. Die Kugel liegt allerdings lediglich über eine Anlagelinie an der Schulter an Je nach Ausgestaltung kann die Kugel aus einem resorbierbarem Material sein.

Zur Sicherung gegen ein Herausziehen des Sehnentransplantates aus der Bohrung wird zusätzlich eine Interferenzschraube eingedreht, die außerdem die Kugel gegen die Schulter am Ende des ersten Abschnittes der Bohrung drückt. Erst durch Eindrehen dieser Fixationsschraube wird das Sehnentransplantat im ersten Abschnitt der Bohrung verblockt.

Nachteilig an dieser Ausgestaltung ist, dass zwei Bauelemente, also einmal die Kugel und zum zweiten die Interferenzschraube, benötigt werden um das Sehnentransplantat zu verblocken. Da sich die Längsachse der Interferenzschraube in Längsachse der Bohrung und somit auch in der Längserstreckung des in diesem Abschnitt aufgenommenen Sehnentransplantates erstreckt, steht die Außenseite der Interferenzschraube über einen relativ langen Bereich in unmittelbarem Kontakt mit dem Sehnentransplantat. Es kann beim Eindrehen der Interferenzschraube nicht ausgeschlossen werden, dass das Sehnentransplantat durch deren Außengewinde verletzt wird, was zu einem Riss des Sehnentransplantates bei Belastung führen kann.

Aus der DE 41 06 823 C1 ist ein spreizbarer Knochendübel zur Fadenfixierung bekannt. Zum Spreizen ist in den Dübelkörper ein Spreizkonus einfahrbar, der mit dem Dübelkörper über eine Sollbruchstelle verbunden ist.

Aus der US 5,632,748 ist eine Vorrichtung zum seitlichen Fixieren eines in einer Bohrung in einem Knochen eingeschobenen Bandes beschrieben. Dazu weist ein Ankerelement an seiner Außenseite längs verlaufende Nuten auf, in die das zu verpressende Implantat eingelegt werden kann. Zur Erhöhung des radialen Anpressdruckes ist vorgesehen, dass das Ankerelement aus zwei Keilen aufgebaut ist, die relativ zueinander verschoben werden können.

Es ist daher Aufgabe der vorliegenden Erfindung hier Abhilfe zu schaffen und eine Vorrichtung zum Verblocken eines Sehnentransplantates zu schaffen, die einfacher aufgebaut ist und keine Beeinträchtigung für das Sehnentransplantat selbst darstellt.

Erfindungsgemäß wird die Aufgabe dadurch gelöst, dass die zwei keilartigen Körper, über eine Sollbruchstelle miteinander verbunden sind, wobei die Vorrichtung beim Gebrauch in eine Bohrung einbringbar ist, die einen ersten Abschnitt mit einem ersten Durchmesser aufweist, in der das Sehnentransplantat aufnehmbar ist, wobei sich daran ein zweiter durchmessergeringerer Abschnitt anschließt, durch den mit dem Sehnentransplantat verbindbare Durchzugsfäden bis zur Außenseite des Knochens reichbar sind, wobei das Element am Einführende des Sehnenimplantates anbringbar ist und an einer Schulter zwischen erstem und zweitem Abschnitt der Bohrung zur Anlage bringbar ist, und dass die radiale Spreizbarkeit durch ein Ziehen an zumindest einem Zugfaden bewirkbar ist.

Die Verblockung erfolgt nunmehr durch das Element selbst, und zwar dadurch, dass es radial spreizbar ausgebildet ist. Das Element ist mit dem Einzugsende des Sehnentransplantates verbunden und kann außerdem mit den Durchzugfäden verbunden sein, und/oder es kann mit separaten Zugfäden versehen sein, die nur mit dem Element verbunden sind und die ausschließlich dem Spreizen dienen.

Das Sehnentransplantat und das radial spreizbare Element und deren Zugfäden werden in die Bohrung eingefädelt, wobei die Zugfäden durch den durchmessergeringeren Abschnitt hindurchgefädelt werden und zur Außenseite reichen. Durch Ziehen an den Zugfäden wird das Element bis an die Schulter zwischen ersten durchmessergrößeren Abschnitt und zweiten durchmessergeringeren Abschnitt herangeführt. Durch ein weiteres Ziehen an den Zugfäden wird nunmehr das Element radial gespreizt und wird dadurch in der Bohrung verblockt. Das gespreizte Element sitzt nunmehr unverrückbar in der Bohrung und, da ja das Sehnentransplantat mit dem Element verbunden ist, ist auch das Sehnentransplantat in der Bohrung verblockt.

Dabei kann die radiale Spreizbarkeit so ausgestaltet sein, dass wenn das Element einmal gespreizt ist, dieses auf Dauer gespreizt bleibt, oder es kann sich auch um eine reversible Spreizbarkeit handeln, d.h. wenn die Spannung bzw. die Zugkraft an den Zugfäden weggenommen wird, kann sich das Element wieder radial zusammenziehen und die Verblockung ist wieder gelöst.

Diese letztere Eigenschaft kann bspw. dann erwünscht sein, wenn nach einem Spannen der Zugfäden auf Grund festgestellter Fehlausrichtung oder Defekte ein anderer Sitz oder ggf. ein anderes Sehnentransplantat eingesetzt werden soll. Es ist erwünscht, dass das Sehnentransplantat in den Knochen einwächst und dadurch auf Dauer fixiert ist. In diesem Fall kann auch vorgesehen sein, dass das Element aus bioresorbierbarem Material hergestellt ist.

Durch die Verschiebung der Keile in axialer Richtung, wobei das einfach durch ein Ziehen an den Zugfäden bewerkstelligt wird, erfolgt die radiale Spreizung des Körpers, wobei das Maß der Spreizung durch das Maß der Bewegung der Keile zueinander gewählt werden kann. Durch diese keilartigen Körper kann eine besonders effektive Verblockung bewerkstelligt werden.

Ein zylindrischer Körper kann über einen sehr großflächigen Bereich an der Schulter zwischen dem Übergang vom ersten durchmessergrößeren Abschnitt zum zweiten durchmessergeringeren Abschnitt anliegen. Die Außenseite der beiden keilartigen Körper ist zylindrisch, so dass dann beim radialen Spreizen durch Verschieben der beiden keilartigen Körper diese über einen großen Flächenbereich an der Innenwand der Bohrung zum Anliegen kommen. Dadurch kann eine besonders effektive, über einen großen Flächenanlagebereich wirkende, Verblockung bewerkstelligt werden.

Die Sollbruchstelle hat den Vorteil, dass der zylindrische Körper vor dem Spreizen als Einheit vorliegt. Dies erleichtert erheblich die Handhabung, bspw. während des Verknotens mit den Zugfäden und das Anbringen an dem Sehnentransplantat und dem anschließenden Einführen und Einfädeln in die Bohrung. Durch Ziehen an den Zugfäden wird dann ein Keil gegenüber dem anderen Keil derart verschoben, dass die radiale Spreizbarkeit bewerkstelligt wird. Diese Zugkraft ist ausreichend, um die Sollbruchstelle zu brechen, wonach die Verschiebbarkeit der keilartigen Körper möglich ist.

In einer weiteren Ausgestaltung ist an den aneinander vorbeigleitenden Keilflächen der keilartigen Körper zumindest eine Raste vorgesehen.

Die Raste ist so ausgebildet, dass ein Vorbeigleiten der Keilflächen in der einen Richtung, bei der die Keile radial gespreizt werden, möglich ist, ein Zurückgleiten aber gesperrt ist.

Dadurch wird eine besonders effektive unverrückbare Verblockung der Keile bewerkstelligt.

Diese Ausgestaltung erlaubt dem Operateur bspw. zunächst den Zusammenbau aus Sehnentransplantat, Keilkörper und Zugfäden an Ort und Stelle zu bringen den Sitz zu überprüfen und dann durch Ziehen an den Zugfäden die Keile nach und nach relativ zueinander zu verschieben, wobei dies stufenweise über mehrere Rastzähne erfolgen kann, so dass durch ein nach und nach immer stärkeres Ziehen die entsprechend feste Verblockung bewerkstelligt werden kann. Die Raste sorgt dann dafür, dass dieser Vorgang irreversibel ist.

In einer weiteren Ausgestaltung der Erfindung weist der Körper an seiner Außenseite Krallen auf, die sich in gespreiztem Zustand des Elements mit der Innenwand des ersten Abschnitts der Bohrung verkrallen.

Diese Maßnahme hat den Vorteil, dass bei der Spreizung des Körpers sich die Krallen an dessen Außenseite in die Wand der Bohrung hineinfressen oder verkrallen, so dass eine besonders gegen Abziehen des Sehnentransplantats wirksame Verblockung bzw. Verkrallung bewerkstelligt werden kann.

In einer weiteren Ausgestaltung der Erfindung ist vorgesehen, dass im Element eine durchgehende Bohrung vorhanden ist, durch die die Zugfäden hindurchführbar sind.

Diese Maßnahme hat den Vorteil, dass die Zugfäden durch die Bohrung des Elements hindurchgeführt werden können, so dass die Außenseite des Elementes voll zur Verblockung, also zum Eingriff mit der Wand der Bohrung zur Verfügung steht.

In einer weiteren Ausgestaltung der Erfindung werden die Zugfäden zum radialen Spreizen des Elements durch die Durchzugfäden des Sehnentransplantates gebildet.

Diese Maßnahme hat den Vorteil, dass die ohnehin am Sehnentransplantat vorhandenen Durchzugfäden zugleich als Zugfäden zum Spreizen des Elements herangezogen werden. Wie zuvor erwähnt, können diese, wenn eine mittige durchgehende Öffnung im Element vorhanden ist, zunächst durchgefädelt werden, so dass dann das Sehnentransplantat an einer Seite des Elementes anliegt. Durch Ziehen an den Durchzugfäden wird dann der Spreizvorgang bewerkstelligt.

Wie bereits erwähnt, können auch separate Zugfäden am Element vorgesehen oder angebracht werden, die ausschließlich dazu dienen, das Element zu spreizen.

In einer weiteren Ausgestaltung der Erfindung besteht das radial spreizbare Element aus Metall, insbesondere aus Titan.

Diese Maßnahme hat den Vorteil, dass ein mechanisch stabiles Element vorliegt, das auf Dauer im gespreizten Zustand im Körper verbleiben kann.

In einer weiteren Ausgestaltung der Erfindung besteht das Element aus einem resorbierbaren Material, insbesondere einem resorbierbaren Polymer.

Diese Maßnahme hat den Vorteil, dass das Sehnentransplantat nach und nach in den Knochen einwachsen kann, wobei der Körper des spreizbaren Elements nach und nach durch nachgewachsenes Knochenmaterial ersetzt wird.

Es versteht sich, dass die vorstehend genannten und die nachstehend noch zu erläuternden Merkmale nicht nur in der jeweils angegebenen Kombination, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: eine perspektivische Ansicht eines ersten Ausführungsbeispiels eines erfindungsgemäßen Elementes zum Verblocken eines Sehnentransplan- tates, das aus zwei keilförmigen Körpern zusammengesetzt ist, und zwar vor dem Spreizen,
- Fig. 2: eine der Fig. 1 vergleichbare perspektivische Ansicht nach dem radialen Spreizen des Elementes durch axiales Verschieben der keilförmigen Körper zueinander,
- Fig. 3: eine Draufsicht auf das Element von Fig. 1 in nicht gespreiztem Zu- stand,
- Fig. 4: eine Draufsicht auf das Element von Fig. 2 in gespreiztem Zustand,
- Fig. 5: eine teilweise geschnittene Ansicht einer Vorrichtung zum Verblocken eines Sehnentransplantates mit dem in Fig. 1 dargestellten Element, wobei der Zusammenbau aus Sehnentransplantatelement und Durch- zugfäden gerade in eine Bohrung eingesetzt ist, noch vor dem radialen Spreizen,
- Fig. 6: einen der Darstellung von Fig. 5 entsprechenden Schnitt nach radialem Spreizen des Elementes entsprechend dem Übergang von Fig. 1 zu Fig. 2, und
- Fig. 7: eine stark vergrößerte ausschnittsweise Darstellung einer Ausgestaltung der aneinander vorbeigleitenden Keilflächen mit einer Raste.

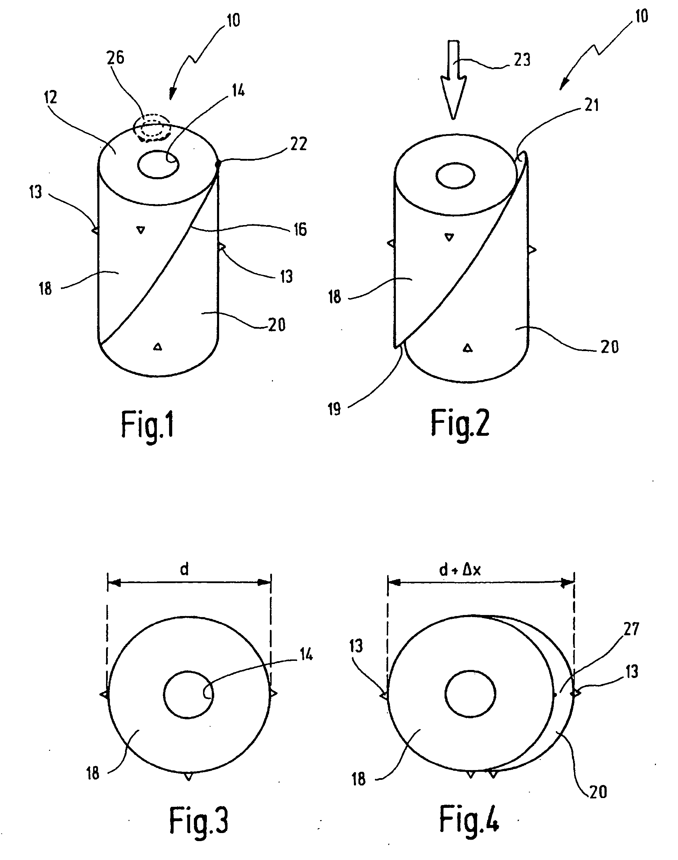

Ein in den Fig. 1 bis 4 dargestelltes radial spreizbares Element ist in seiner Gesamtheit mit der Bezugsziffer 10 bezeichnet.

Das Element 10 besteht aus einem zylindrischen Körper 12, der mittig eine durchgehende Bohrung 14 aufweist.

Der Körper 12 ist über einen diagonalen Schrägschnitt 16 in zwei keilförmige Körper 18 und 20 aufgeteilt. Die Schnittfläche definiert zugleich die beiden gegenüberliegenden Keilflächen 19 und 21 der keilförmigen Körper 18 und 20.

Der Schrägschnitt 16 ist nicht vollkommen durchgehend ausgeführt, sondern die beiden keilförmigen Körper 18 und 20 sind noch über eine etwa punktförmige Sollbruchstelle 22 miteinander verbunden, so dass der Körper 12 in der Darstellung von Fig. 1 gehalten ist, d.h. die beiden keilförmigen Körper 18 und 20 sich nicht voneinander trennen. An seiner Außenseite ist der Körper 12, mit von diesem radial vorstehenden Vorsprüngen in Form von Krallen 13 versehen. Aus der Draufsicht von Fig. 3 ist zu erkennen, dass der Körper einen Durchmesser d aufweist.

Wird auf den keilförmigen Körper 18 eine Kraft in Richtung eines Pfeiles 23 ausgeübt und ruht der zweite keilförmige Körper 20 auf einem Widerlager verschiebt sich der keilförmige Körper 18, nach Bruch der Sollbruchstelle 22, in Richtung des Pfeiles 23.

Diese Kraft in Richtung des Pfeiles 23 kann durch eine Zugkraft ausgeübt werden, wie das nachfolgend noch näher erläutert wird, z.B. durch einen mit dem keilförmigen Körper 18 verbundenen Zugfäden. Die Verbindung kann durch eine am Körper 18 vorgesehene Öse 26 erfolgen.

Dadurch wird der Körper 12 in radialer Richtung gesehen gespreizt, wobei dies insbesondere aus Fig. 4 ersichtlich ist. Das heißt, dass radiale Maß d hat sich um den Betrag Δx vergrößert, der abhängig davon ist, wie weit die beiden keilförmigen Körper 18 und 20 relativ zueinander verschoben werden.

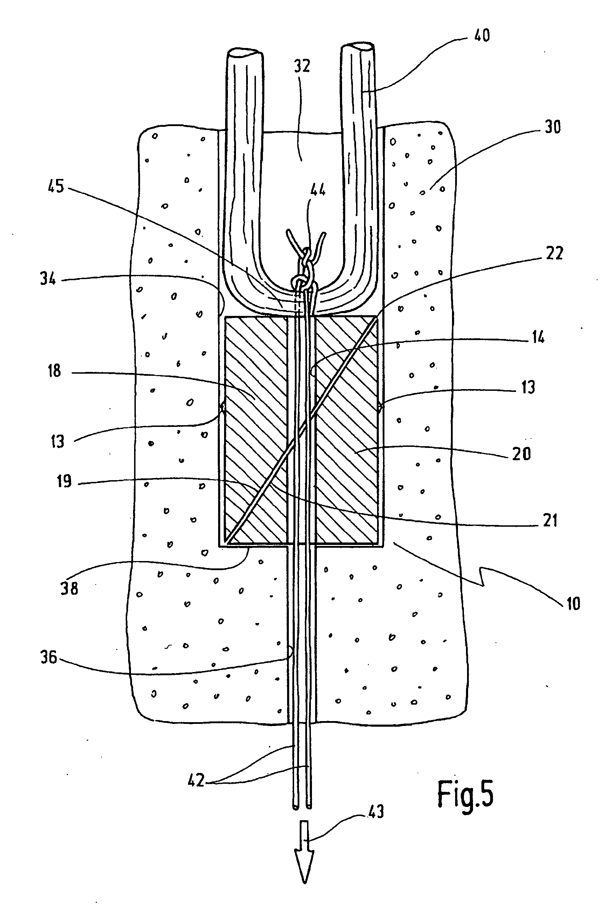

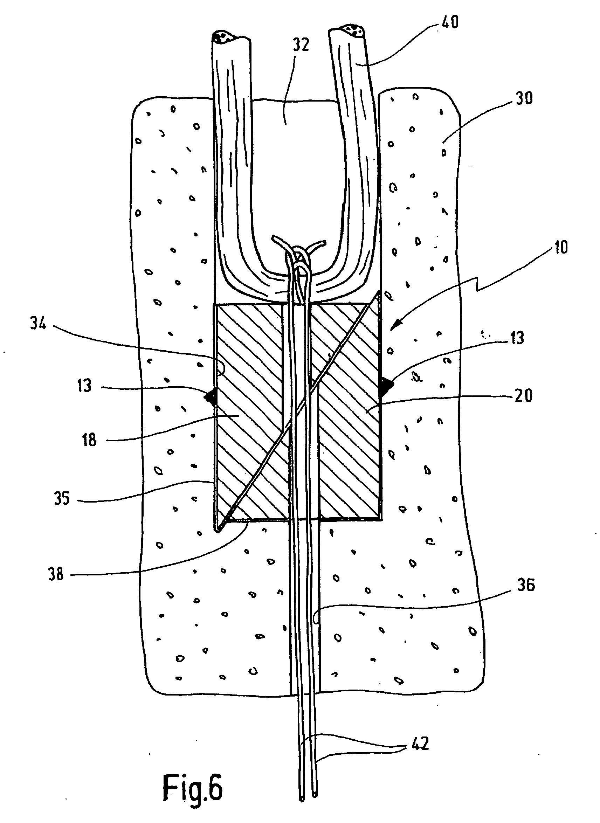

In den Fig. 5 und 6 ist nunmehr dargestellt, wie diese Verschiebung der beiden keilförmigen Körper 18 und 20 bewerkstelligt werden kann.

Es ist ersichtlich, dass in einem Knochen 30, bspw. im Oberschenkelknochen eine Bohrung 32 eingebracht ist, die einen ersten Abschnitt 34 mit einem ersten Durchmesser aufweist, der in einen zweiten Abschnitt 36 mit einem geringeren Durchmesser übergeht, wobei der zweite Abschnitt 36 bis zur Außenseite des Knochens am Oberschenkel reicht. Am Übergang zwischen erstem Abschnitt 34 und zweitem Abschnitt 36 ist eine Schulter 38 ausgebildet.

Der lichte Innendurchmesser des ersten Abschnitts 34 entspricht etwa dem Außendurchmesser d des Elementes 10 von Fig. 1 inklusive dessen von der Außenseite abstehender Krallen 13.

Ein Sehnentransplantat 40, das in der Bohrung 32 verankert und verblockt werden soll, wird üblicherweise an seinem Einführende 45 zu einer U-förmigen Schleife geformt.

Bei dem Sehnentransplantat 40 kann sich es um ein künstliches Transplantat oder auch um eine natürliche Sehne handeln, die dem Patienten an einer anderer Stelle entnommen wurde bspw. die Semitendinosussehne.

Das Einführende 45 des Sehnentransplantates 40 wird mit Durchzugfäden 42 verknotet und das Element 10 wird auf das Einführende 45 aufgefädelt, indem die Durchzugfäden 42 durch die mittige durchgehende Öffnung 14 im Körper 12 durchgefädelt werden und das Element 10 über die Durchzugfäden 42 bis an das Einführende 45 herangeschoben wird.

Wenn gewünscht, kann durch eine zusätzliche Verknotung das Element 10 mit dem Sehnentransplantat 40 verbunden werden. Es kann in einer weiteren Ausführungsform ein separater Zugfaden vorgesehen sein, der nur mit dem oberen keilförmigen Körper 18 verbunden ist, z.B. mit diesem verknotet ist, wozu an diesem eine entsprechende Öse 26 (siehe Fig. 1) vorhanden ist. Das Spreizen der keilförmigen Körper 18 und 20 kann dann nur durch diesen Zugfaden bewerkstelligt werden.

Diese Vorrichtung aus Sehnentransplantat 40, Durchzugfäden 42 und Element 10 wird nun in die Bohrung 32 eingeschoben, und zwar mit den Durchzugfäden 42 voran, die durch den zweiten Abschnitt 36 hindurchgefädelt werden und bis zur Außenseite reichen. Durch Ziehen an den Durchzugfäden 42 wird der Zusammenbau so weit in die Bohrung 32 eingezogen, bis die voreilende Stirnseite des Elementes 10 auf der Schulter 38 zum Liegen kommt, wie das in Fig. 5 ersichtlich ist. In dieser Position kann vom Operateur der korrekte Sitz überprüft werden.

Zum Verblocken wird nun kräftig an den Durchzugfäden 42 in Richtung des in Fig. 5 dargestellten Pfeiles 43 gezogen. Dadurch wirkt auf den keilförmigen Körper 18 eine Kraft ein. Da der andere keilförmige Körper 20 auf der Schulter 38 als Widerlager anliegt, gleitet der keilförmige Körper 18 längs der Keilflächen 19 und 21 etwas nach unten, wobei, wie zuvor im Übergang von Fig. 3 zu Fig. 4 beschrieben, das Element 10 radial gespreizt wird.

Diese Situation nach dem Spreizen ist in Fig. 6 dargestellt. Bei der Relativverschiebung der keilförmigen Körper 18 und 20 wird das Element 10 in dem ersten Abschnitt 34 der Bohrung 32 verblockt, wobei diese Verblockung dauernd dadurch aufrecht erhalten wird, dass die Durchzugfäden 42 in diesem gespannten Zustand gehalten werden. Dies wird dadurch bewerkstelligt, dass die Durchzugfäden 42 an der Außenseite mit einem sogenannten Fixationsbutton verknotet werden, wobei durch ein nachträgliches Verdrehen dieses Fixationsbuttons diese Spannung noch eingestellt werden kann. Gleiches gilt für die Ausführungsform mit dem separaten Zugfaden.

Bei dem Spreizen des Elementes 10 durch die Relativbewegung der keilförmigen Körper 18 und 20 fressen sich zusätzlich die an der Außenseite des Elements 10 vorhandenen Krallen 13 in die Innenwand 35 der Bohrung 32 hinein, also in die Spongiosa des Knochens 30. Dadurch wird eine zusätzliche Abzugssicherung geschaffen. Die zuvor erwähnte Zugkraft ist ausreichend, dass die Sollbruchstelle 22, die die beiden keilförmigen Körper 18 und 20 miteinander verbindet, bricht, wonach die Verschiebemöglichkeit gegeben ist.

Bei der in den Fig. 1 bis 6 beschriebenen Ausgestaltung des Elementes 10 sind die gegenüberliegenden Keilflächen 19 und 21 glatt, so dass bei Wegnehmen der Zugspannung an den Durchzugfäden 22 die beiden keilförmigen Körper 18 und 20 theoretisch wieder in die in Fig. 5 beschriebene Lage bewegt werden könnten. Falls nach dem Setzen und dem Verblocken festgestellt wird, dass das Sehnentransplantat schadhaft ist und sofort noch am Operationsort durch ein anderes ersetzt werden muss, so kann diese Ausgestaltung gewünscht sein.

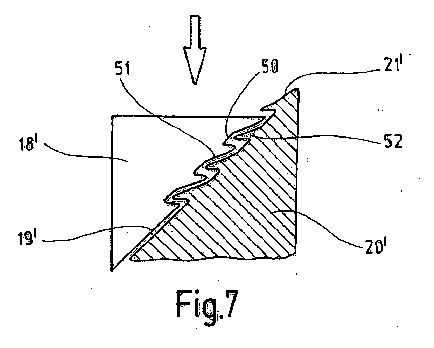

In Fig. 7 ist eine weitere Ausgestaltung dargestellt, wobei ersichtlicht ist, dass an den gegenüberliegenden Keilflächen 19' und 21' von zwei ansonst gleich wie zuvor beschrieben ausgebildeten keilförmigen Körpern 18' und 20' eine Raste 50 vorgesehen ist. Die jeweiligen Zähne 51 bzw. 52 an den gegenüberliegenden keilförmigen Körpern 18' und 20' sind so ausgebildet, dass eine Relativverschiebung wie zuvor beschrieben unter Spreizung des Körpers 12 möglich ist, indem die Zähne 51, 52 übereinander gleiten, in entgegengesetzter Richtung jedoch diese Bewegung gesperrt ist.

In dieser Ausgestaltung ist dann die Verschiebung irreversibel, was zu einer unverrückbaren festsitzenden Verblockung führt.

## Patentansprüche

1. Vorrichtung zum Verblocken eines Sehnentransplantates (40) in einer Bohrung (32) in einem Knochen, mit einem Element (10), das beim Gebrauch in die Bohrung (32) einbringbar ist, wobei das Element (10) zwei keilartige Körper (18, 20, 18', 20') aufweist, die in axialer Richtung zueinander verschiebbar sind, wodurch das Element radial spreizbar ist, wobei das Element (10), vor einem Spreizen, etwa zylindrisch ausgebildet ist und das Element (10) über einen Schrägschnitt in zwei keilartige Körper (18, 20, 18', 20') aufgeteilt ist, wobei die Vorrichtung beim Gebrauch in eine Bohrung (32) einbringbar ist, die einen ersten Abschnitt (34) mit einem ersten Durchmesser aufweist, in der das Sehnentransplantat (40) aufnehmbar ist, wobei sich daran ein zweiter durchmessergeringerer Abschnitt (36) anschließt, durch den mit dem Sehnentransplantat (40) verbindbare Durchzugsfäden (42) bis zur Außenseite des Knochens (30) reichbar sind, wobei das Element (10) am Einführende (45) des Sehnenimplantates (40) anbringbar ist und an einer Schulter (38) zwischen erstem (34) und zweitem Abschnitt (36) der Bohrung (32) zur Anlage bringbar ist, und dass die radiale Spreizbarkeit durch ein Ziehen an zumindest einem Zugfaden (42) bewirkbar ist, **dadurch gekennzeichnet, dass** die zwei keilartigen Körper (18, 20, 18', 20') über eine Sollbruchstelle (22) miteinander verbunden sind.

2. Vorrichtung nach Anspruch 1, **dadurch gekennzeichnet, dass** an den aneinander vorbeigleitenden Keilflächen (19', 21') der keilförmigen Körper (18', 20') zumindest eine Raste (50) vorgesehen ist.

3. Vorrichtung nach Anspruch 1 oder 2, **dadurch gekennzeichnet, dass** der Körper (12) an seiner Außenseite Krallen (13) aufweist, die sich in gespreiztem Zustand des Elementes (10) mit einer Innenwand des ersten Abschnittes der Bohrung (32) verkrallen.

4. Vorrichtung nach einem der Ansprüche 1 bis 3, **dadurch gekennzeichnet, dass** im Element (10) eine durchgehende Bohrung (14) vorhanden ist, durch die die Zugfäden (42) hindurchführbar sind.

5. Vorrichtung nach einem der Ansprüche 1 bis 4, **dadurch gekennzeichnet, dass** der zumindest eine Zugfaden zum radialen Spreizen des Elementes durch die Durchzugfäden (42) des Sehnentransplantates (40) gebildet ist.

6. Vorrichtung nach einem der Ansprüche 1 bis 5, **dadurch gekennzeichnet, dass** das Element (10) aus metallischem Material, insbesondere aus Titan, hergestellt ist.

7. Vorrichtung nach einem der Ansprüche 1 bis 5, **dadurch gekennzeichnet, dass** das Element (10) aus resorbierbarem Material, insbesondere aus einem resorbierbaren Polymer, hergestellt ist.

## Claims

1. Device for blocking a tendon graft (40) in a bore (32) in a bone, with an element (10) which can be inserted during use into the bore (32), wherein the element (10) has two wedge-shaped bodies (18, 20, 18', 20') which can be displaced relative to one another in an axial direction, thereby radially expanding the element, wherein the element (10), prior to expanding, is approximately cylindrical, and wherein the element (10) is divided by an oblique cut into two wedge-shaped bodies (18, 20, 18', 20'), wherein the device can be inserted during use into a bore (32) having a first section (34) with a first diameter, into which the tendon graft (40) can be housed and which is followed by a second section (36) of smaller diameter through which pulling-threads (42) which are connectable to the tendon graft (40) can pass up to the outer side of the bone (30), wherein the element (10) can be mounted to an insertion end (45) of the tendon graft (40) and can rest against a shoulder (38) between first (34) and second section (36) of the bore (32), and wherein the radial expansion can be made by pulling at at least one pulling-thread (42), **characterized in that** the two wedge-like bodies (18, 20, 18', 20') are connected via a predetermined break point (22).

2. Device of claim 1, **characterized in that** at least one catch (50) is provided on the wedge surfaces (19', 21') of the wedge-shaped body (18', 20') that slide along one another.

3. Device of claims 1 or 2, **characterized in that** the body (12) has, on its outer face, claws (13) which engage with an inner wall of said first section of said drilled bore (32), when said element (10) is in an expanded state.

4. Device of anyone of claims 1 through 3, **characterized in that** the element (10) has a continuous bore (14) through which the pulling-threads (42) can be guided.

5. Device of anyone of claims 1 through 4, **characterized in that** the at least one pulling-thread for radial expansion of the element is formed by the pulling-threads (42) of said tendon graft (40).

6. Device of anyone of claims 1 through 5, **characterized in that** the element (10) is made of a metallic material, in particular from titanium.

7. Device of anyone of claims 1 through 5, **characterized in that** the element (10) is made of an absorbable material, in particular of an absorbable polymer.

## Revendications

1. Dispositif pour fixer un greffon de tendon (40) dans un perçage (32) dans un os, avec un élément (10) qui peut être introduit, lors de son utilisation, dans le perçage (32), l'élément (10) présentant deux corps en forme de coin (18, 20, 18', 20') qui sont mobiles l'un par rapport à l'autre en direction axiale, grâce à quoi l'élément est extensible radialement, l'élément (10) étant, avant une extension, de forme approximativement cylindrique et l'élément (10) étant divisé en deux corps en forme de coin (18, 20, 18', 20') par une coupe diagonale, le dispositif pouvant être introduit, lors de son utilisation, dans un perçage (32) qui présente une première portion (34) ayant un premier diamètre, dans laquelle le greffon de tendon (40) peut être logé, à laquelle fait suite une deuxième portion (36) de plus petit diamètre à travers laquelle des fils de traction (42) pouvant être reliés au greffon de tendon (40) peuvent s'étendre jusqu'à la face extérieure de l'os (30), l'élément (10) pouvant être placé à l'extrémité d'introduction (45) du greffon de tendon (40) et mis en application contre un épaulement (38) entre la première (34) et la deuxième portion (36) du perçage (32), l'extensibilité radiale étant obtenue par une traction sur au moins un fil de traction (42), **caractérisé en ce que** les deux corps en forme de coin (18, 20, 18', 20') sont reliés ensemble par une zone de rupture (22).

2. Dispositif selon la revendication 1, **caractérisé en ce qu'**au moins un cran d'arrêt (50) est prévu sur les surfaces en coin (19', 21') glissant le long l'une de l'autre des corps en forme de coin (18', 20').

3. Dispositif selon la revendication 1 ou 2, **caractérisé en ce que** le corps (12) présente sur sa face extérieure des crampons (13) qui, dans l'état étendu de l'élément (10), se cramponnent à une paroi intérieure de la première portion du perçage (32).

4. Dispositif selon une des revendications 1 à 3, **caractérisé en ce qu'**un perçage traversant (14), à travers lequel les fils de traction (42) peuvent être passés, est présent dans l'élément (10).

5. Dispositif selon une des revendications 1 à 4, **caractérisé en ce que** ledit au moins un fil de traction pour l'extension radiale de l'élément est formé par les fils de traction (42) du greffon de tendon (40).

6. Dispositif selon une des revendications 1 à 5, **caractérisé en ce que** l'élément (10) est fabriqué à partir d'un matériau métallique, en particulier de titane.

7. Dispositif selon une des revendications 1 à 5, **caractérisé en ce que** l'élément (10) est fabriqué à partir d'un matériau résorbable, en particulier d'un polymère résorbable.
